# EUROPEAN PATENT APPLICATION

(11) **EP 1 953 553 A2**
(43) Date of publication of application: **06.08.2008**
(21) Application number: 08001802.1
(22) Date of filing: 31.01.2008
(51) Int. Cl.: G01N 33/543

(54) **Biosensor substrate**

(30) Priority: 01.02.2007 JP 2007022594; 19.03.2007 JP 2007070049
(71) Applicant: FUJIFILM Corporation, Minato-ku Tokyo (JP)
(72) Inventor: Saitoh, Yukou, Ashigarakami-gu Kanagawa 258-8577 (JP); Ezoe, Toshihide, Woodbridge, CT 06525 (US); Nishimi, Taisei, Ashigarakami-gu Kanagawa 258-8577 (JP)
(74) Representative: HOFFMANN EITLE

(57) **Abstract**

Biosensor substrate that prevents non-specific adsorption and used for immobilizing a physiologically active substance. The biosensor substrate comprises an inorganic oxide film and a hydrophilic polymer having a physiologically active substance-immobilizing group on the substrate, wherein the hydrophilic polymer is bound to the inorganic oxide film through a low molecular compound.

## Description

### Technical Field

The present invention relates to a biosensor substrate that prevents non-specific adsorption and is used for analyzing biomolecular interaction.

### Background Art

Recently, a large number of measurements using intermolecular interactions such as immune responses are being carried out in clinical tests, etc. However, since conventional methods require complicated operations or labeling substances, several techniques are used that are capable of detecting the change in the binding amount of a test substance with high sensitivity without using such labeling substances. Examples of such a technique may include a surface plasmon resonance (SPR) measurement technique, a quartz crystal microbalance (QCM) measurement technique, and a measurement technique of using functional surfaces ranging from gold colloid particles to ultra-fine particles. The SPR measurement technique is a method of measuring changes in the refractive index near an organic functional film attached to the metal film of a chip by measuring a peak shift in the wavelength of reflected light, or changes in amounts of reflected light in a certain wavelength, so as to detect adsorption and desorption occurring near the surface. The QCM measurement technique is a technique of detecting adsorbed or desorbed mass at the ng level, using a change in frequency of a crystal due to adsorption or desorption of a substance on gold electrodes of a quartz crystal (device). In addition, the ultra-fine particle surface (nm level) of gold is functionalized, and physiologically active substances are immobilized thereon. Thus, a reaction to recognize specificity among physiologically active substances is carried out, thereby detecting a substance associated with a living organism from sedimentation of gold fine particles or sequences. The measurement technology of detecting refractive index changes using a waveguide is a technology of detecting an effective refractive index change of a medium adjacent to the waveguide by optical change.

In all of the above-described techniques, the surface where a physiologically active substance is immobilized is important. Surface plasmon resonance (SPR), which is most commonly used in this technical field, will be described below as an example.

A commonly used measurement chip comprises a transparent substrate (e.g., glass), an evaporated metal film, and a thin film having thereon a functional group capable of immobilizing a physiologically active substance. The measurement chip immobilizes the physiologically active substance on the metal surface via the functional group. A specific binding reaction between the physiological active substance and a test substance is measured, so as to analyze an interaction between biomolecules.

National Publication of International Patent Application No. 2003-516519 discloses a biosensor substrate of which metal or metal oxide surface has a multifunctional copolymer that is electrostatically immobilized thereto. In such a substrate, the copolymer that can bind to a physiologically active substance is allowed to be immobilized to substrates of various different types of materials. However, the pH at which the substrate is used is restricted, and the copolymer may be detached from the substrate. National Publication of International Patent Application No. 2004-507580 discloses an ionic polymer surface to which a hydrophilic monomer graft polymer is bound. Since the hydrophilic polymer is covalently bound to the substrate, the hydrophilic polymer is not detached. However, the non-specific adsorption inhibition property of the substrate surface is not sufficient. In Japanese Patent No. 2815120, there is disclosed to introduce a carboxyl group into dextran by binding dextran to a gold substrate through a self-assembled membrane (SAM) and further repeating the reaction with bromoacetic acid. This protein-immobilizing membrane has a three dimensional matrix and therefore has a merit of enabling to immobilize a large amount of protein. However, since dextran is immobilized on the substrate through a thiol molecule, the material that can be used as the substrate surface is limited to free electron metals. Accordingly, the application of an optical sensor is restricted.

### Disclosure of the Invention

An object of the present invention is to solve the above-mentioned problems associated with conventional techniques. That is, it is an object of the present invention to provide a biosensor substrate that prevents non-specific adsorption and is used for immobilizing a physiologically active substance. Furthermore, it is an object of the present invention to provide a biosensor substrate that can be imparted with transparency.

The present inventors have conducted intensive studies to overcome the above-mentioned problems. As a result, they have found a fact that a desired biosensor substrate can be provided by binding a hydrophilic polymer that can immobilize a physiologically active substance to the surface made of an inorganic oxide film on the substrate through a low molecular compound. Thus, the present invention has been completed.

That is, the present invention provides a biosensor substrate which comprises an inorganic oxide film and a hydrophilic polymer having a physiologically active substance-immobilizing group on the substrate, wherein the hydrophilic polymer is bound to the inorganic oxide film through a low molecular compound. The low molecular compound preferably has a molecular weight of 100 to 500.

The binding is preferably a covalent bond.

The low molecular compound is preferably a compound represented by the following formula:

X-L(Y)ₙ

wherein X represents a functional group that can bind to an inorganic oxide and is selected from the group consisting of -Si(OCH₃)₃, -Si(OC₂H₅)₃, -Si(OC₃H₇)₃, and -Si(OC₄H₉)₃; Y represents a functional group that binds to a hydrophilic polymer and is selected from the group consisting of -OH, -SH, -COOH, -NH₂, -NR¹R² (wherein R¹ and R² each independently represent a hydrogen atom or a lower alkyl group), -CHO, a glycidoxy group, a methacryloxy group, a mercapto group, a vinyl group, and an epoxy group; when n is not 1, Y's may be the same or different; L represents a single bond or a group selected from the group consisting of an alkylene group, a phenylene group, NH-, -CO-NH-, -O-, -S-, polyethylene oxide, polypropylene oxide, fatty acid ester, and a combination thereof, and may include -OH, -SH, -COOH, -NH₂, -NR¹R² (wherein R¹ and R² each independently represent a hydrogen atom or a lower alkyl group), or -CHO in the molecule; and n represents an integer of 1 to 5.

The inorganic oxide is preferably selected from the group consisting of silicon oxide, titanium oxide, zirconium oxide, and a mixture thereof.

The inorganic oxide film preferably has a thickness of 0.1 to 500 nm.

The inorganic oxide film is formed on a layer of gold, silver, or platinum.

The physiologically active substance-immobilizing group is preferably selected from the group consisting of -OH, -SH, -COOH, -NR¹R² (wherein R¹ and R² each independently represent a hydrogen atom or a lower alkyl group), -CHO, -NR³NR¹R² (wherein R¹, R², and R³ each independently represent a hydrogen atom or a lower alkyl group) -NCO, -NCS, an epoxy group, a vinyl group, a biotinyl group, biotin-binding protein, digoxigenin, and an anti-digoxigenin antibody.

The present invention further provides a biosensor which comprises the above-mentioned biosensor substrate.

The biosensor substrate according to the present invention prevents non-specific adsorption and is used for immobilizing a physiologically active substance thereon. Furthermore, the biosensor substrate can be imparted with transparency.

### Brief Description of the Invention

Figure 1 is a perspective view of the entire biosensor according to an embodiment.
Figure 2 is a perspective view of a sensor stick according to the embodiment.
Figure 3 is an exploded perspective view of the sensor stick according to the embodiment.
Figure 4 is a cross-sectional view of one liquid channel portion of the sensor stick according to the embodiment.
Figure 5 is a diagram illustrating a state in which light beams enter the measurement region and the reference region of the sensor stick according to the embodiment.
Figure 6 is a perspective view of a pipette according to the embodiment.
Figure 7 is a schematic view of the vicinity of the optical measurement portion of the biosensor according to the embodiment.
Figure 8 is a schematic block diagram of the controller and the vicinity thereof according to the embodiment.

### Preferred Embodiments of the Invention

Hereinafter, the embodiments of the present invention will be explained.

The biosensor in the present invention has as broad a meaning as possible, and the term biosensor is used herein to mean a sensor, which converts an interaction between biomolecules into a signal such as an electric signal, so as to measure or detect a target substance. The conventional biosensor is comprised of a receptor site for recognizing a chemical substance as a detection target and a transducer site for converting a physical change or chemical change generated at the site into an electric signal. In a living body, there exist substances having an affinity with each other, such as enzyme/substrate, enzyme/coenzyme, antigen/antibody, or hormone/receptor. The biosensor operates on the principle that a substance having an affinity with another substance, as described above, is immobilized on a substrate to be used as a molecule-recognizing substance, so that the corresponding substance can be selectively measured.

In the biosensor according to the present invention, a film of an inorganic oxide is employed. Examples of the inorganic oxide include SiO₂, TiO₂, ZnO₂, SnO₂, Ta₂O₂, HfO₂, and Sc₂O₃. These oxides can be used alone or in a combination thereof. In addition, the oxide may be used as a mixture with another material such as Si₃N₄. Among such oxides, SiO₂, TiO₂, ZnO₂, and a mixture thereof can reduce non-specific adsorption of physiologically active substances and substances having affinity to physiologically active substances and, therefore, are particularly preferred as biosensor surface materials.

The thickness of the inorganic oxide film is not limited. For example, a thickness between 0.1 nm and 500 nm is preferred, and a thickness between 1 nm and 200 nm is particularly preferred.

The inorganic oxide film may be formed according to a common method. For example, sputtering, vapor deposition, ion plating, electroplating, non-electroplating, adhesion of a film, or press-bonding of a film can be conducted.

For example, in a surface plasmon resonance biosensor, it is preferable that the above-mentioned inorganic oxide film be adjacent to a layer of a material that exhibits surface plasmon resonance directly or through an intermediate layer. Examples of the material exhibiting surface plasmon resonance preferably include free electron metals such as gold, silver, copper, aluminum, and platinum, and gold is particularly preferred. These metals can be used alone or in a combination thereof. Furthermore, an interposition layer of chromium or the like may be disposed between the substrate and the layer of a metal, in consideration for adhesion to the substrate.

The film thickness of a metal is not limited. When the metal film is used for a surface plasmon resonance biosensor, the thickness is preferably between 0.1 nm and 500 nm, and particularly preferably between 1 nm and 200 nm. If the thickness exceeds 500 nm, the surface plasmon phenomenon of a medium cannot be sufficiently detected. Moreover, when an interstitial layer consisting of chrome or the like is provided, the thickness of the interstitial layer is preferably between 0.1 nm and 10 nm.

Formation of a metal film may be carried out by common methods, and examples of such a method may include sputtering method, evaporation method, ion plating method, electroplating method, nonelectrolytic plating method, adhesion of a film, and press-bonding of a film.

The inorganic oxide film is preferably disposed on the substrate. Here, the term "disposed on the substrate" means not only that the inorganic oxide film is disposed so as to be directly contacted with the substrate, but also that the inorganic oxide film is disposed via another layer without being directly contact with the substrate. For example, as a substrate that can be used in the present invention in a case of a surface plasmon resonance biosensor, the above-mentioned inorganic oxide film is preferably disposed on an optical substrate through a layer of a material that can exhibit surface plasmon resonance. In general, the optical substrate is made of a material transparent to a laser beam, for example, optical glass such as BK7 or a synthetic resin, more specifically, polymethylmethacrylate, polyethylene terephthalate, polycarbonate, or a cycloolefin polymer. Such a substrate is preferably made of a material that is not anisotropic to polarized light and has excellent workability. In a substrate used in a measurement technique for detecting a change in refractive index using a waveguide, the substrate is preferably made of a material having a low refractive index to the waveguide for transmitting a laser light beam used for detection through the inorganic oxide layer as the waveguide. The substrate material is preferably made of a material transparent to a laser beam, for example, optical glass or a synthetic resin, more specifically, polymethylmethacrylate, polyethylene terephthalate, polycarbonate, or a cycloolefin polymer.

In the present invention, a hydrophilic polymer is bound to an inorganic oxide layer through a low molecular compound. The low molecular compound preferably has a molecular weight of 100 to 500. In general, compounds called silane coupling agents are preferred as the low molecular compound.

Examples of the silane coupling agents that can be used in the present invention include compounds represented by the following formula:

X-L(Y)ₙ

wherein X represents a functional group that can bind to an inorganic oxide and is selected from the group consisting of -Si(OCH₃)₃, -Si(OC₂H₅)₃, -Si(OC₃H₇)₃, and -Si(OC₄H₉)₃; Y represents a functional group that binds to a hydrophilic polymer and is selected from the group consisting of -OH, -SH, -COOH, -NH₂, -NR¹R² (wherein R¹ and R² each independently represent a hydrogen atom or a lower alkyl group), -CHO, a glycidoxy group, a methacryloxy group, a mercapto group, a vinyl group, and an epoxy group; when n is not 1, Y's may be the same or different; L represents a single bond or a group selected from the group consisting of an alkylene group, a phenylene group, -NH-, -CO-NH-, -O-, -S-, polyethylene oxide, polypropylene oxide, fatty acid ester, and a combination thereof and may includes -OH, -SH, -COOH, -NH₂, -NR¹R² (wherein R¹ and R² each independently represent a hydrogen atom or a lower alkyl group), or -CHO in the molecule; and n represents an integer of 1 to 5.

The silane coupling agent reacts with water to generate a silanol group by hydrolysis and forms a covalent bond with an inorganic oxide surface or a metal surface by an acid-base reaction. Examples of the silane coupling agent are as follows:

H2C=CH-S i (-O-C H3)3

H2C=CH-S i (-O-C H2-C H3)3

H2C=CH-CH2-S i (-O-CH3)3

H2C=CH-CH2-S i (-O-CH2-CH3)3

H2C=CH-CH2-CH2-CH2-CH2-CH2-CH2-S i(-O-CH3)3

H2C=CH-C6H4-CH2-NH-CH2-CH2-NH-CH2-S i (-O-C H3)3

H2N-CH2-CH2-CH2-S i(-O-CH2-CH3)3

H2N-CO-NH-CH2-CH2-CH2-S i(-O-CH3)3

H2N-CH2-CH2-NH-CH2-CH2-NH-CH2-CH2-S i (-O-C H3)3

H S-CH2-CH2-CH2-S i (-O-CH3)3

C 1 -CH2-CH2-CH2-S i (-O-CH3)3

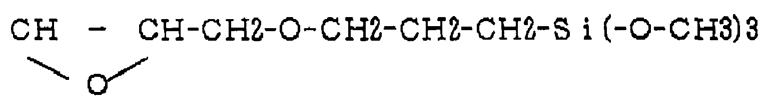

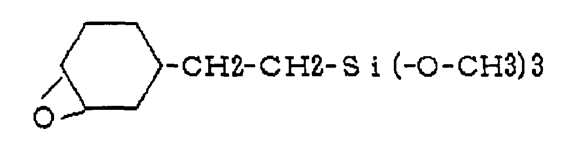

HO-CO-CH2-CH2-S-CH2-CH2-CH2-S i (-O-CH3)3

HO-CO-CH2-NH-CH2-NH-CH2-CH2-CH2-S i (-O-CH3)3

HO-CO-CH=CH-CO-NH-CH2-CH2-CH2-S i (-O-CH2-CH3)3

Among them, preferred examples are as follows:

H2C=CH-CH2-S i (-O-CH3)3

H2C=CH-CH2-S i(-O-CH2-CH3)3

H2N-CH2-CH2-CH2-S i (-O-CH2-CH3)3

H2N-CO-NH-CH2-CH2-CH2-S i (-O-CH3)3

HS-CH2-CH2-CH2-S i (-O-CH3)3

C 1-CH2-CH2-CH2-S i (-O-CH3)3

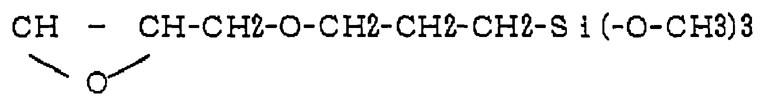

The surface of the sensor used in the present invention is modified with a hydrophilic polymer compound. Examples of the hydrophilic polymer compound include protein such as albumin and casein; sugar derivatives such as agar, sodium alginate, and starch derivatives; cellulose compounds such as carboxymethylcellulose and hydroxymethylcellulose; polysaccharides such as dextran, dextran derivatives, chitin, and chitosan; and synthetic hydrophilic polymers such as polyvinyl alcohol, poly-N-vinyl pyrroridone, polyacrylamide, and polyacrylic acid.

The hydrophilic polymer compound can be coated on a substrate according to a common method, for example, by spin coating, air knife coating, bar coating, blade coating, slide coating, or curtain coating. Furthermore, a spray method, a vapor deposition method, a cast method, or a dipping method can be employed. In addition, the hydrophilic polymer compound may be chemically bound to a substrate through a molecule that is chemically bound to the substrate surface.

The hydrophilic polymer compound immobilized to the sensor surface preferably has a thickness of 1 nm to 300 nm in an aqueous solution. Thinner film thickness results in a smaller amount of a physiologically active substance being immobilized. In addition, since a hydrate layer on the sensor surface is thinned, the physiologically active substance itself is denatured to make it difficult to detect the interaction with an analyte. Thicker film thickness results in an obstacle of diffusion of an analyte in the film. In particular, when interaction is detected from the opposite side of the hydrophilic polymer compound-immobilizing face of a sensor substrate, the distance between the detection surface and the interaction-forming portion is large to cause a decrease in detection sensitivity. The thickness of a hydrophilic polymer compound in an aqueous solution can be evaluated by, for example, AFM or ellipsometry.

These polymer materials can be surface-modified by chemical treatment with, for example, a chemical agent, a coupling agent, a surfactant or surface vapor deposition or physical treatment with, for example, heat, ultraviolet, a radiation, plasma, or ions.

The hydrophilic polymer compound preferably has a functional group that can generate a covalent bond with a physiologically active substance. Examples of the functional group that can generate a covalent bond with a physiologically active substance include -OH, -SH, -COOH, -NR¹R² (wherein R¹ and R² each independently represent a hydrogen atom or a lower alkyl group), -CHO, -NR³NR¹R² (wherein R¹, R², and R³ each independently represent a hydrogen atom or a lower alkyl group), -NCO, -NCS, an epoxy group, and a vinyl group. Here, the number of carbon in the lower alkyl group is not particularly limited and is generally about C 1 to C10 and preferably C 1 to C6.

In a case that a polymer containing a carboxyl group is used as the hydrophilic polymer compound, the hydrophilic polymer compound can be immobilized to a substrate coated with a self-assembled membrane by activating the carboxyl group. The polymer containing a carboxyl group can be activated by a known method, for example, a method of activating with, water-soluble carbodiimides, 1-(3-dimethylaminopmpyl)-3 ethylcarbodiimide (EDC) and N-hydroxysuccinimide (NHS) or a method of activating with EDC alone. The biosensor according to the present invention can be produced by treating the polymer containing a thus activated carboxyl group with a substrate having an amino group.

Moreover, another method for activation of a polymer containing a carboxyl groups is a method that uses a nitrogen-containing compound. Specifically, a nitrogen-containing compound represented by the following formula (Ia) or (Ib) [wherein R₁ and R₂ mutually independently denote a carbonyl group, a carbon atom, or a nitrogen atom, which may have a substituent, R₁ and R₂ may form 5- to 6-membered rings via binding, "A" denotes a carbon atom or a phosphorus atom, which has a substituent, "M" denotes an (n-1)-valent element, and "X" denotes a halogen atom] can also be used.

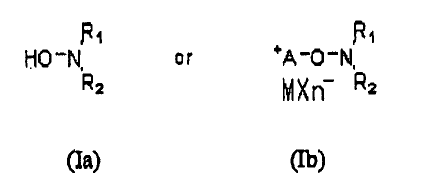

R₁ and R₂ mutually independently denote a carbonyl group, a carbon atom, or a nitrogen atom, which may have a substituent, and preferably R₁ and R₂ form 5- to 6-membered rings via binding. Particularly preferably, hydroxysuccinic acid, hydroxyphthalic acid, 1-hydroxybenzotriazole, 3,4-dihydroxy-3-hydroxy-4-oxo-1,2,3-benzotriazine, and a derivative thereof are provided.

Further preferably, a nitrogen-containing compound represented by the following compound can also be used.

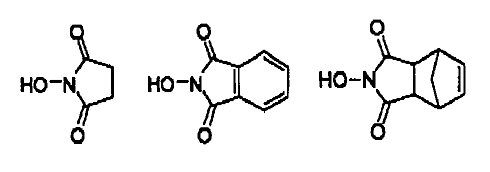

More preferably, a compound represented by the following formula (II) [wherein "Y" and "Z" mutually independently denote CH or a nitrogen atom] can also be used as a nitrogen-containing compound.

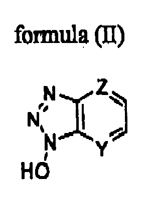

Specifically, the following compounds can be used, for example,

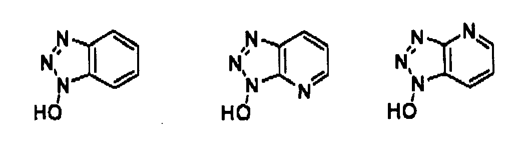

Further preferably, the following compound can also be used as a nitrogen-containing compound.

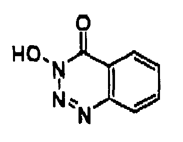

Further preferably, a compound represented by the following formula (III) [wherein "A" denotes a carbon atom or a phosphorus atom, which has a substituent, "Y" and "Z" mutually independently denote CH or a nitrogen atom, "M" denotes a (n-1)-valent element, and X denotes a halogen atom] can also be used as a nitrogen-containing compound.

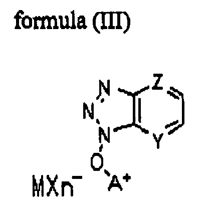

A substituent of a carbon atom or a phosphorus atom denoted by "A" is preferably an amino group having a substituent. Furthermore, a dialkylamino group such as a dimethylamino group or a pyrrolidino group is preferable. Examples of an (n-1)-valent element denoted by "M" include a phosphorus atom, a boron atom, and an arsenic atom. A preferable example of such (n-1)-valent element is a phosphorus atom. Examples of a halogen atom denoted by "X" include a fluorine atom, a chlorine atom, a bromine atom, and an iodine atom. A preferable example of such halogen atom is a fluorine atom.

Furthermore, specific examples of such nitrogen-containing compound represented by formula (III) include the following compounds, for example.

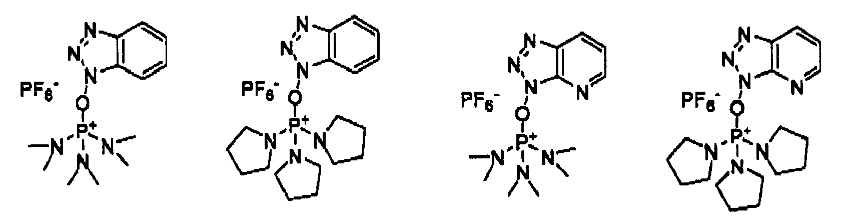

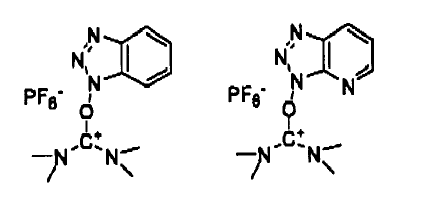

Further preferably, a compound represented by the following formula (IV) [wherein "A" denotes a carbon atom or a phosphorus atom, which has a substituent, "M" denotes an (n-1)-valent element, and "X" denotes a halogen atom] can also be used as a nitrogen-containing compound.

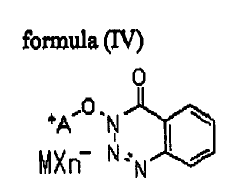

Specifically, the following compound can be used, for example.

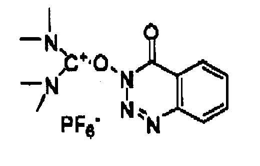

Moreover, as a method for activating a polymer containing carboxyl group, the use of a phenol derivative having an electron-withdrawing group is also preferable. Furthermore, the σ value of the electron-withdrawing group is preferably 0.3 or higher. Specifically, the following compounds or the like can also be used.

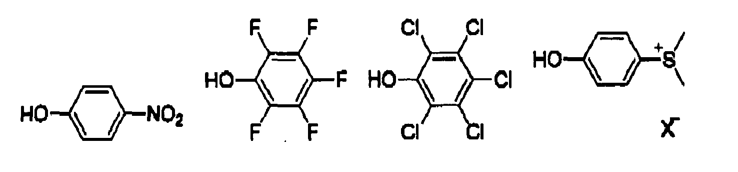

Furthermore, a carbodiimide derivative can further be used separately for such method for activating a polymer containing carboxyl group in combination with the above compounds. Preferably, a water-soluble carbodiimide derivative can be used in combination with such compounds. Further preferably, the following compound (1-Ethyl-3-(3-dimethylaminopropyl)carbodiimide, hydrochloride) can be used in combination with such compounds.

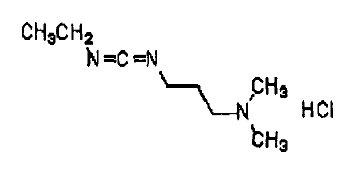

The above carbodiimide derivative and nitrogen-containing compound or phenol derivative can be used not only in such manner, but also independently, if desired. Preferably, a carbodiimide derivative and a nitrogen-containing compound are used in combination.

Furthermore, the following compound can also be used in such method for activating carboxyl groups. The compound can be used independently and can also be used in combination with a carbodiimide derivative, a nitrogen-containing compound, and/or a phenol derivative.

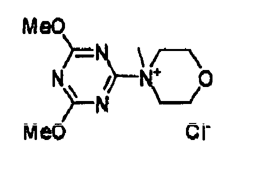

As a method of activating carboxylic acid in the polymer containing a carboxyl group, the method described in Japanese Patent Application No. 2004-238396 (JP Patent Publication (Kokai) No.2006-58071A), paragraphs [0011] to [0022] (that is, a method of activating a carboxyl group existing on the surface of a substrate using any compound selected from a uronium salt, a phosphonium salt, and a triazine derivative, which have a specific structure, so as to form a carboxylic amide group) and the method described in Japanese Patent Application No. 2004-275012 (JP Patent Publication (Kokai) No.2006-90781A), paragraphs [0011] to [0019] (that is, a method, which comprises activating a carboxyl group existing on the surface of a substrate using a carbodiimide derivative or a salt thereof, converting the resultant to an ester using any compound selected from a nitrogen-containing hetero aromatic compound having a hydroxyl group, a phenol derivative having an electron attracting group, and an aromatic compound having a thiol group, and allowing the ester to react with amine, so as to form a carboxylic amide group) can preferably be used.

It is to be noted that the aforementioned uronium salt, phosphonium salt, and triazine derivative, which have a specific structure, described in Japanese Patent Application No. 2004-238396(JP Patent Publication (Kokai) No.2006-58071A), mean the uronium salt represented by the following formula 1, the phosphonium salt represented by the following formula 2, and the triazine derivative represented by the following formula 3, respectively. 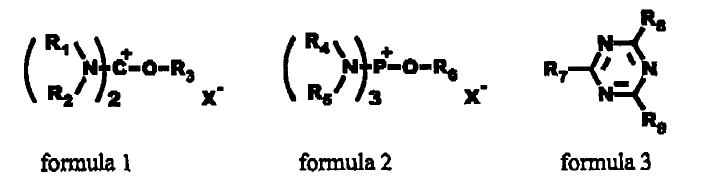 (in formula 1, each of R₁ and R₂ independently represents an alkyl group containing 1 to 6 carbon atoms, or R₁ and R₂ together form an alkylene group containing 2 to 6 carbon atoms, which forms a ring together with an N atom, R₃ represents an aromatic ring group containing 6 to 20 carbon atoms, or a hetero ring group containing at least one heteroatom, and X⁻ represents an anion; in formula 2, each of R₄ and R₅ independently represents an alkyl group containing 1 to 6 carbon atoms, or R₄ and R₅ together form an alkylene group containing 2 to 6 carbon atoms, which forms a ring together with an N atom, R₆ represents an aromatic ring group containing 6 to 20 carbon atoms, or a hetero ring group containing at least one heteroatom, and X⁻ represents an anion; and in formula 3, R₇ represents an onium group, and each of R₈ and R₉ independently represents an electron donating group.)

Furthermore, it is also preferable that, in the hydrophilic polymer compound coating layer, a ligand that binds to a tag molecule with a Kd (binding constant) of 10⁻¹² M or less is used as a linkage group for a physiologically active substance. Combination of a tag molecule/a ligand is, for example, biotin/biotin-binding protein, digoxigenin/an anti-digoxigenin antibody, or a D-Ala-D-Ala derivative/a vancomycin trimer derivative described in SCIENCE, 280, 708-711 (1998), but is not limited thereto. Examples of the biotin-biding protein include avidins (such as avidin, streptavidin, or variants thereof).

Furthermore, after the formation of a thin film of a hydrophilic polymer compound, the substrate surface may be chemically modified so that a physiologically active substance can be immobilized thereto. As a result, advantageously, an analyte can be measured or detected by converting biomolecular interaction into a signal such as an electrical signal. This chemical modification can be conducted by a common method for introducing a functional group that can generate a covalent bond between a substrate surface and a physiologically active substance in an aqueous solution or an organic solvent.

The reaction group introduced into a polymer as a group for immobilizing a physiologically active substance may be a carboxyl group or an amino group. Also, a state is also possible in which a physiologically active substance such as biotin-binding protein (such as avidin, streptavidin, or neutravidin), Protein A, Protein G, an antigen, or an antibody (for example, a known tag antibody such as an anti-GST antibody) is previously immobilized. A physiologically active substance having a membrane structure such as a lipid, can be immobilized by using an immobilization layer of a polymer to which alkane is introduced. According to an application purpose, the length of a polymer chain, the thickness of a polymer, the density of a polymer, or the amount of a reaction group to be introduced into a polymer is controlled. As a result, it is possible to be applied to various types of protein. Furthermore, a His-tag ligand or the like can be immobilized through a metal chelate by introducing NTA (nitrilotriacetic acid) or the like as an immobilization group into a polymer.

In the biosensor surface obtained as mentioned above, a physiologically active substance can be immobilized on a metal surface or metal film by covalently binding the physiologically active via the aforementioned functional group.

A physiologically active substance immobilized on the sensor substrate of the present invention is not particularly limited, as long as it interacts with a measurement target. Examples of such a substance may include an immune protein, an enzyme, a microorganism, nucleic acid, a low molecular weight organic compound, a nonimmune protein, an immunoglobulin-binding protein, a sugar-binding protein, a sugar chain recognizing sugar, fatty acid or fatty acid ester, and polypeptide or oligopeptide having a ligand-binding ability.

Examples of an immune protein may include an antibody whose antigen is a measurement target, and a hapten. Examples of such an antibody may include various immunoglobulins such as IgG, IgM, IgA, IgE or IgD. More specifically, when a measurement target is human serum albumin, an anti-human serum albumin antibody can be used as an antibody. When an antigen is an agricultural chemical, pesticide, methicillin-resistant *Staphylococcus aureus,* antibiotic, narcotic drug, cocaine, heroin, crack or the like, there can be used, for example, an anti-atrazine antibody, anti-kanamycin antibody, anti-metamphetamine antibody, or antibodies against O antigens 26, 86, 55, 111 and 157 among enteropathogenic *Escherichia coli.*

An enzyme used as a physiologically active substance herein is not particularly limited, as long as it exhibits an activity to a measurement target or substance metabolized from the measurement target. Various enzymes such as oxidoreductase, hydrolase, isomerase, lyase or synthetase can be used. More specifically, when a measurement target is glucose, glucose oxidase is used, and when a measurement target is cholesterol, cholesterol oxidase is used Moreover, when a measurement target is an agricultural chemical, pesticide, methicillin-resistant *Staphylococcus aureus,* antibiotic, narcotic drug, cocaine, heroin, crack or the like, enzymes such as acetylcholine esterase, catecholamine esterase, noradrenalin esterase or dopamine esterase, which show a specific reaction with a substance metabolized from the above measurement target, can be used.

A microorganism used as a physiologically active substance herein is not particularly limited, and various microorganisms such as *Escherichia coli* can be used.

As nucleic acid, those complementarily hybridizing with nucleic acid as a measurement target can be used. Either DNA (including cDNA) or RNA can be used as nucleic acid. The type of DNA is not particularly limited, and any of native DNA, recombinant DNA produced by gene recombination and chemically synthesized DNA may be used.

As a low molecular weight organic compound, any given compound that can be synthesized by a common method of synthesizing an organic compound can be used.

A nonimmune protein used herein is not particularly limited, and examples of such a nonimmune protein may include avidin (streptoavidin), biotin, and a receptor. Examples of an immunoglobulin-binding protein used herein may include protein A, protein G, and a rheumatoid factor (RF).

As a sugar-binding protein, for example, lectin is used.

Examples of fatty acid or fatty acid ester may include stearic acid, arachidic acid, behenic acid, ethyl stearate, ethyl arachidate, and ethyl behenate.

Preferably, a solution that contains a physiologically active substance is applied onto the surface of a substrate where a hydrophilic polymer compound is immobilized, and the solution is then dried, so as to immobilize the physiologically active substance.

With regard to the concentration of a solution (an applied solution) that contains physiologically active substance, it is preferable that the concentration of physiologically active substance immobilized on a substrate surface be high. The aforementioned concentration is preferably between 0.1 mg/ml and 10 mg/ml, and more preferably between 1 mg/ml and 10 mg/ml, although it depends on the type of physiologically active substance.

In the process of drying such a solution that contains physiologically active substance, the physiologically active substance tend to be precipitated from the peripheral portion of the applied solution, or from a portion in which the liquid remains immediately before the applied solution is dried. Thereby, the quantities of physiologically active substance immobilized on the substrate surface vary. This is not preferable. In order to uniform the quantities of physiologically active substance immobilized on the substrate surface, it is preferable that the viscosity of the applied solution be set at high, so far as it does not inhibit the binding of the physiologically active substance to the substrate surface. By setting the viscosity of the applied solution at high, the movement of the physiologically active substance contained in the applied solution in the horizontal direction towards the substrate surface can be suppressed during the drying process. As a result, variations in the quantities of physiologically active substance immobilized can be suppressed. The viscosity of the applied solution is preferably maintained at 0.9 cP or more during the drying process.

The term "drying process" is used in the present invention to mean natural drying whereby after application of a solution that contains a physiologically active substances, the solution is left at rest, or an intentional drying process whereby the speed of drying the aforementioned solution is increased by heating or sir-blowing. An increase in the drying rate of the solution that contains the physiologically active substance (coating solution) is also effective for suppressing variation in the amount of the physiologically active substance immobilized. The drying rate is increased, and the drying process is sufficiently rapidly completed with respect to the movement of the physiologically active substance in the horizontal direction. Thereby, the drying process is completed before the physiologically active substance substantially moves, so that the variation can be suppressed. When such a coating solution contains water, a high drying rate can be obtained by drying the coating solution in an environment wherein a difference between a dry-bulb temperature and a, wet-bulb temperature is great. The coating solution is dried in an environment wherein a temperature difference between the dry-bulb temperature and the wet-bulb temperature is preferably 7°C or greater, and more preferably 10°C or greater, further preferably 13.5°C or greater. In addition, considering the production process, the drying time is preferably 10 minutes or shorter, more preferably 5 minutes or shorter, and particularly preferably 1 minute or shorter.

An example of a method of applying a solution that contains physiologically active substance is a method particularly using a dispenser that quantitatively discharges a solution to be applied. The discharge port of the dispenser is moved on a substrate at a certain speed at certain intervals, so that the solution can be uniformly applied at any given sites on the substrate. When the solution is applied using a dispenser, the interval between the substrate and the discharge port is extremely narrowed, and the thickness of the applied solution is reduced, so that the thickness of physiologically active substance can be uniformed. Further, the drying speed can also be increased. Thus, the use of such a dispenser is preferable. Another preferred method of applying a solution that contains physiologically active substance is spin coating. This method is particularly preferably applied when the thickness of the applied film is reduced. In this method, since the drying process is carried out after a solution having a uniformed thickness has been formed, it is preferable to prevent evaporation of the solution during rotation of a spin coater. If a method of placing a substrate in a hermetically sealed vessel or the like during rotation is applied to maintain the concentration of a solvent existing around the substrate at high, the drying speed can be controlled before and after formation of a thin film during rotation. Thus, this method is particularly preferable. It is preferred that drying is carried out under a condition where temperature and humidity are kept constant, after this coating process.

When the interaction of physiologically active substance with test substances is detected, variations in the quantities of physiologically active substance immobilized on the sensor surface cause an error in quantitative and kinetic evaluation of such an interaction. In order to keep such an error to a minimum, the quantities of physiologically active substance immobilized are preferably uniformed. A CV value (coefficient variation) (standard deviation/mean value), which indicates variations in the quantities of physiologically active substance immobilized on the surface of a substrate used in detection of an interaction, is preferably 15% or less, and more preferably 10% or less. Such a CV value can be calculated based on the quantities of physiologically active substance immobilized on at least two sites, preferably 10 or more sites, and more preferably 100 or more sites on the substrate surface. Uniformity can be evaluated by quantifying the quantities of substances existing on a sensor substrate before and after immobilization of physiologically active substance. However, such uniformity can also be evaluated by fluorescently-labeling substances that have been known to bind to physiologically active substance, immobilizing such fluoreseently-labeled substances on a sensor substrate, and then measuring fluorescence intensity using a fluorescence microscope or the like. Moreover, it is also possible to quantify physiologically active substance using an SPR imager, an ellipsometer, TOF-SEMS, an ATR-IR apparatus, etc.

In the present invention, a compound having a residue capable of forming hydrogen bond (hereinafter referred to as Compound S) may be used for improving storage stability of a physiologically active substance which was immobilized on a substrate.

Generally, physiologically active substance such as protein maintain its three-dimensional structure by coordination of water molecules in a solution, but when it is dried, physiologically active substance cannot maintain its three-dimensional structure and is denatured. Further, physiologically active substance is contained in a hydrophilic polymer compound on a surface of substrate, physiologically active substance aggregate by drying, and aggregates are produced. The compound S having a residue capable of forming hydrogen bond which may be used in the present invention can be used for the purpose of suppressing denature of physiologically active substance by maintaining the three-dimensional structure in place of water or suppressing the aggregation by steric effect by covering the physiologically active substance.

In the present invention, the polymer having a residue capable of forming hydrogen bond is preferably added as a aqueous solution to a layer on substrate where physiologically active substance was immobilized. polymer can be added by coating a mixed solution of polymer and physiologically active substance on a surface of substrate, or by immobilizing physiologically active substances on a surface of substrate and then over-coating the polymer. When a mixed solution of polymer and physiologically active substance is coated, fluctuation of the amount of immobilized physiologically active substances can be reduced. Preferably, an aqueous solution of polymer can be added to substrate in a state of thin film. A method for forming thin film on substrate may be any known method. Examples thereof include extrusion coating, curtain coating, casting, screen printing, spin coating, spray coating, slidebead coating, slit and spin coating, slit coating, die coating, dip coating, knife coating, blade coating, flow coating, roll coating, wire-bar coating, and transferring printing. In the present invention, spray coating or spin coating is preferably used, and spin coating is more preferably used as a method for forming a thin film on substrate, since a coated film having a controlled film thickness can be easily prepared.

The concentration of the applied solution of compound S is not particularly limited, as long as it does not cause a problem regarding permeation into a layer that contains physiologically active substances. The aforementioned concentration is preferably between 0.1% by weight and 5% by weight. In addition, in terms of applicability and regulation of pH, a surfactant, a buffer, an organic solvent, a salt may also be added to the applied solution.

The compound S having a residue capable of forming hydrogen bond is preferably a compound which is non-volatile under normal pressure at normal temperature. The average molecular weight of the compound is preferably 350 to 5,000,000, more preferably 1,200 to 2,000,000, most preferably 1,200 to 70,000. The compound S having a hydroxyl group in molecule is preferably saccharide. The saccharide may be monosaccharide or .polysaccharide. In case of n-saccharide, n is preferably 4 to 1,200, and n is more preferably 20 to 600.

If the mean molecular weight of compound S is too low, the compound is crystallized on the surface of a substrate. This causes disruption of a hydrophilic polymer layer, on which physiologically active substances are immobilized, and disruption of the three-dimensional structure of the physiologically active substances. In contrast, if the mean molecular weight of compound S is too high, it causes problems such that it impairs immobilization of physiologically active substances on a substrate, that a layer that contains physiologically active substances cannot be impregnated with compound S, and that layer separation occurs.

For the purpose of suppressing degradation of physiologically active substances immobilized on a substrate, the aforementioned compound S having a residue capable of forming hydrogen bond preferably has a dextran skeleton or a polyethylene oxide skeleton. The type of a substituent used is not limited, as long as the object of the present invention can be achieved. Moreover, for the purpose of suppressing degradation of physiologically active substances immobilized on a substrate, a nonionic compound having no dissociable groups is preferably used as compound S. Furthermore, the aforementioned compound S having a residue capable of forming a hydrogen bond preferably has high affinity for water molecules. A distribution coefficient LogP value between water and n-octanol is preferably 1 or greater. Such LogP value can be measured by the method described in Japanese Industrial Standard (JIS), Z7260-107 (2000), "Measurement of distribution coefficient (1-octanol/water) - Shaking method," etc.

Specific examples of compound S having a residue capable of forming hydrogen bond include: compounds consisting of two or more types of residues selected from polyalcohols such as polyvinyl alcohol, proteins such as collagen, gelatin, or albumin, polysaccharides such as hyaluronic acid, chitin, chitosan, starch, cellulose, alginic acid, or dextran, polyethers including polyethyleneoxy-polypropylene oxide condensates such as polyethylene glycol, polyethylene oxide, polypropylene glycol, polypropylene oxide, or Pluronic, Tween 20, Tween 40, Tween 60, Tween 80, etc.; and derivatives and polymers of such compounds. Of these, polysaccharides and polyethers are preferable, and polysaccharides are more preferable. Specifically, dextran, cellulose, Tween 20, Tween 40, Tween 60, and Tween 80 are preferably used. Further, a nonvolatile monomer and a nonvolatile water-soluble oligomer described in JP Patent Publication (Kokai) No. 2006-170832 A can also be used. Examples of such a nonvolatile monomer used herein may include: tetrose, pentose, heptose and hextose, wherein a hydroxyl group may be protected by a protecting group, and their glycoside; methyl glucoside; and cyclitols, wherein a hydroxyl group may be protected by a protecting group. Moreover, examples of such a nonvolatile water-soluble oligomer include: an oligomer represented by general formula (1) (-[CH2-CH(CONH2)-]n-), general formula (2) (-[CH2-CH2-O-]n-), or general formula (3) (-[CH2-CH(OH)-]n-) (wherein, in general formulas (1) to (3), n represents an integer between 10 and 200); and an oligosaccharide having an n number of sugars (2 ≤ n ≤ 10), wherein a hydroxyl group may be protected by a protecting group. Furthermore, sugars described in US 2003/0175827 and DE 20306476A1, such as trehalose, sucrose, maltose, lactose, xylitol, fructose, mannitol, glucose, xylol, maltodextran, saccharose, or polyvinylpyrrolidone may also be used. It is preferable that such compound S be substantially identical to the basic skeleton of a hydrophilic polymer used in the present invention. The term "basic skeleton" is used herein to mean a ring structure of sugar, for example. Although the type of a functional group or length differs, if such a ring structure is identical, it is considered that the basic skeleton is substantially identical.

A biosensor to which a physiologically active substance is immobilized as described above can be used to detect and/or measure a substance which interacts with the physiologically active substance.

In the present invention, it is preferable to detect and/or measure an interaction between a physiologically active substance immobilized on the substrate and a test substance by a nonelectric chemical method. Examples of a non-electrochemical method may include a surface plasmon resonance (SPR) measurement technique, a quartz crystal microbalance (QCM) measurement technique, and a measurement technique that uses functional surfaces ranging from gold colloid particles to ultra-fine particles.

In a preferred embodiment of the present invention, the substrate of the present invention can be used as a biosensor for surface plasmon resonance which is characterized in that it comprises a metal film placed on a transparent substrate.

A biosensor for surface plasmon resonance is a biosensor used for a surface plasmon resonance biosensor, meaning a member comprising a portion for transmitting and reflecting light emitted from the sensor and a portion for immobilizing a physiologically active substance. It may be fixed to the main body of the sensor or may be detachable.

The surface plasmon resonance phenomenon occurs due to the fact that the intensity of monochromatic light reflected from the border between an optically transparent substance such as glass and a metal thin film layer depends on the refractive index of a sample located on the outgoing side of the metal. Accordingly, the sample can be analyzed by measuring the intensity of reflected monochromatic light.

A device using a system known as the Kretschmann configuration is an example of a surface plasmon measurement device for analyzing the properties of a substance to be measured using a phenomenon whereby a surface plasmon is excited with a lightwave (for example, Japanese Patent Laid-Open No. 6-167443). The surface plasmon measurement device using the above system basically comprises a dielectric block formed in a prism state, a metal film that is formed on a face of the dielectric block and comes into contact with a measured substance such as a sample solution, a light source for generating a light beam, an optical system for allowing the above light beam to enter the dielectric block at various angles so that total reflection conditions can be obtained at the interface between the dielectric block and the metal film, and a light-detecting means for detecting the state of surface plasmon resonance, that is, the state of attenuated total reflection, by measuring the intensity of the light beam totally reflected at the above interface.

In order to achieve various incident angles as described above, a relatively thin light beam may be caused to enter the above interface while changing an incident angle. Otherwise, a relatively thick light beam may be caused to enter the above interface in a state of convergent light or divergent light, so that the light beam contains components that have entered therein at various angles. In the former case, the light beam whose reflection angle changes depending on the change of the incident angle of the entered light beam can be detected with a small photodetector moving in synchronization with the change of the above reflection angle, or it can also be detected with an area sensor extending along the direction in which the reflection angle is changed. In the latter case, the light beam can be detected with an area sensor extending to a direction capable of receiving all the light beams reflected at various reflection angles.

With regard to a surface plasmon measurement device with the above structure, if a light beam is allowed to enter the metal film at a specific incident angle greater than or equal to a total reflection angle, then an evanescent wave having an electric distribution appears in a measured substance that is in contact with the metal film, and a surface plasmon is excited by this evanescent wave at the interface between the metal film and the measured substance. When the wave vector of the evanescent light is the same as that of a surface plasmon and thus their wave numbers match, they are in a resonance state, and light energy transfers to the surface plasmon. Accordingly, the intensity of totally reflected light is sharply decreased at the interface between the dielectric block and the metal film. This decrease in light intensity is generally detected as a dark line by the above light-detecting means. The above resonance takes place only when the incident beam is p-polarized light. Accordingly, it is necessary to set the light beam in advance such that it enters as p-polarized light.

If the wave number of a surface plasmon is determined from an incident angle causing the attenuated total reflection (ATR), that is, an attenuated total reflection angle (θSP), the dielectric constant of a measured substance can be determined. As described in Japanese Patent Laid-Open No. 11-326194, a light-detecting means in the form of an array is considered to be used for the above type of surface plasmon measurement device in order to measure the attenuated total reflection angle (θSP) with high precision and in a large dynamic range. This light-detecting means comprises multiple photo acceptance units that are arranged in a certain direction, that is, a direction in which different photo acceptance units receive the components of light beams that are totally reflected at various reflection angles at the above interface.

In the above case, there is established a differentiating means for differentiating a photodetection signal outputted from each photo acceptance unit in the above array-form light-detecting means with regard to the direction in which the photo acceptance unit is arranged. An attenuated total reflection angle (θSP) is then specified based on the derivative value outputted from the differentiating means, so that properties associated with the refractive index of a measured substance are determined in many cases.

In addition, a leaking mode measurement device described in "Bunko Kenkyu (Spectral Studies)" Vol. 47, No. 1 (1998), pp. 21 to 23 and 26 to 27 has also been known as an example of measurement devices similar to the above-described device using attenuated total reflection (ATR). This leaking mode measurement device basically comprises a dielectric block formed in a prism state, a clad layer that is formed on a face of the dielectric block, a light wave guide layer that is formed on the clad layer and comes into contact with a sample solution, a light source for generating a light beam, an optical system for allowing the above light beam to enter the dielectric block at various angles so that total reflection conditions can be obtained at the interface between the dielectric block and the clad layer, and a light-detecting means for detecting the excitation state of waveguide mode, that is, the state of attenuated total reflection, by measuring the intensity of the light beam totally reflected at the above interface.

In the leaking mode measurement device with the above structure, if a light beam is caused to enter the clad layer via the dielectric block at an incident angle greater than or equal to a total reflection angle, only light having a specific wave number that has entered at a specific incident angle is transmitted in a waveguide mode into the light wave guide layer, after the light beam has penetrated the clad layer. Thus, when the waveguide mode is excited, almost all forms of incident light are taken into the light wave guide layer, and thereby the state of attenuated total reflection occurs, in which the intensity of the totally reflected light is sharply decreased at the above interface. Since the wave number of a waveguide light depends on the refractive index of a measured substance placed on the light wave guide layer, the refractive index of the measurement substance or the properties of the measured substance associated therewith can be analyzed by determining the above specific incident angle causing the attenuated total reflection.

In this leaking mode measurement device also, the above-described array-form light-detecting means can be used to detect the position of a dark line generated in a reflected light due to attenuated total reflection. In addition, the above-described differentiating means can also be applied in combination with the above means.

The above-described surface plasmon measurement device or leaking mode measurement device may be used in random screening to discover a specific substance binding to a desired sensing substance in the field of research for development of new drugs or the like. In this case, a sensing substance is immobilized as the above-described measured substance on the above thin film layer (which is a metal film in the case of a surface plasmon measurement device, and is a clad layer and a light guide wave layer in the case of a leaking mode measurement device), and a sample solution obtained by dissolving various types of test substance in a solvent is added to the sensing substance. Thereafter, the above-described attenuated total reflection angle (θSP) is measured periodically when a certain period of time has elapsed.

If the test substance contained in the sample solution is bound to the sensing substance, the refractive index of the sensing substance is changed by this binding over time. Accordingly, the above attenuated total reflection angle (θSP) is measured periodically after the elapse of a certain time, and it is determined whether or not a change has occurred in the above attenuated total reflection angle (θSP), so that a binding state between the test substance and the sensing substance is measured. Based on the results, it can be determined whether or not the test substance is a specific substance binding to the sensing substance. Examples of such a combination between a specific substance and a sensing substance may include an antigen and an antibody, and an antibody and an antibody. More specifically, a rabbit anti-human IgG antibody is immobilized as a sensing substance on the surface of a thin film layer, and a human IgG antibody is used as a specific substance.

It is to be noted that in order to measure a binding state between a test substance and a sensing substance, it is not always necessary to detect the angle itself of an attenuated total reflection angle (θSP). For example, a sample solution may be added to a sensing substance, and the amount of an attenuated total reflection angle (θSP) changed thereby may be measured, so that the binding state can be measured based on the magnitude by which the angle has changed. When the above-described array-form light-detecting means and differentiating means are applied to a measurement device using attenuated total reflection, the amount by which a derivative value has changed reflects the amount by which the attenuated total reflection angle (θSP) has changed. Accordingly, based on the amount by which the derivative value has changed, a binding state between a sensing substance and a test substance can be measured (Japanese Patent Application No. 2000-398309 filed by the present applicant). In a measuring method and a measurement device using such attenuated total reflection, a sample solution consisting of a solvent and a test substance is added dropwise to a cup- or petri dish-shaped measurement chip wherein a sensing substance is immobilized on a thin film layer previously formed at the bottom, and then, the above-described amount by which an attenuated total reflection angle (θSP) has changed is measured.

Moreover, Japanese Patent Laid-Open No. 2001-330560 describes a measurement device using attenuated total reflection, which involves successively measuring multiple measurement chips mounted on a turntable or the like, so as to measure many samples in a short time.

When the biosensor of the present invention is used in surface plasmon resonance analysis, it can be applied as a part of various surface plasmon measurement devices described above.

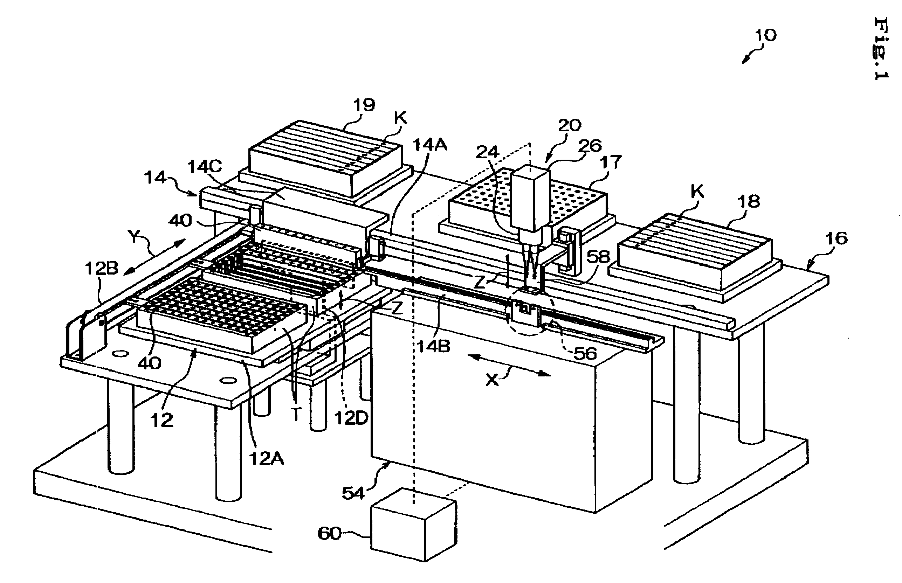

The biosensor of the present invention can be used as a biosensor, which has a waveguide structure on the surface of a carrier, for example, and which detects refractive index changes using such a waveguide. In this case, the waveguide structure on the carrier surface has a diffraction grating, and in some cases, an additional layer. This waveguide structure is a planar waveguide body comprising a thin dielectric layer. Light gathered to the waveguide body form is introduced into such a thin layer by total internal reflection. The transmission velocity of the thus introduced light wave (hereinafter referred to as "mode") is indicated as a C/N value. Herein, C indicates the velocity of light in a vacuum, and N indicates an effective refractive index of the mode introduced into the waveguide body. Such an effective refractive index N is determined based on the structure of the waveguide body on one face, and is determined based on the refractive index of a medium adjacent to the thin waveguide layer on the other face. Conduction of a light wave is carried out not only in a thin planar layer, but also by another waveguide structure, and in particular, by a stripped waveguide body. In such a case, the waveguide structure is processed into the shape of a stripped film. It is an important factor for a biosensor that changes in effective refractive indexes N are generated as a result of changes in the medium adjacent to the waveguide layer, and changes in the refractive index and thickness of the waveguide layer itself or an additional layer adjacent to the waveguide layer. The structure of a biosensor of this system is described in page 4, line 48 to page 14, line 15, and Figures 1 to 8 of JP Patent Publication (Kokoku) No. 6-27703 B (1994).

For example, in one embodiment, there is a structure whereby a waveguide layer comprising a planar thin layer is established on a substrate (e.g. Pyrex (registered trademark) glass). A waveguide layer and a substrate form together a so-called waveguide body. Such a waveguide layer can be a multilayer laminated body such as an oxide layer (SiO2, SnO2, Ta2O5, TiO2, TiO2-SiO2, HfO2, ZrO2, Al2O3, Si3N4, HfON, SiON, scandium oxide, or a mixture thereof) or a plastic layer (e.g. polystyrene, polyethylene, polycarbonate, etc.). For transmission of light into a waveguide layer as a result of total internal reflection, the refractive index of the waveguide layer must be greater than that of the adjacent medium (for example, a substrate, or an additional layer as described later). A diffraction grating is disposed on the surface of the waveguide layer or in the bosom thereof, which faces to a substrate or a measured substance. Such a diffraction grating can be formed in a carrier according to embossing, holography, or other methods. Subsequently, the upper surface of the diffraction grating is coated with a thin waveguide film having a higher refractive index. The diffraction grating has the functions to focus rays of light incident on the waveguide layer, to discharge the mode already introduced into the waveguide layer, or to transmit a portion of the mode in the travel direction and reflect a portion thereof. The grating area of the waveguide layer is covered with an additional layer. Such an additional layer can be a multilayer film, as necessary. This additional layer is able to have the function to carry out selective detection of a substance contained in a measured substance. In a preferred embodiment, a layer having a detection function can be established on the outermost surface of such an additional layer. As such a layer having a detection function, a layer capable of immobilizing physiologically active substances can be used.

In another embodiment, it is also possible to adopt a structure whereby an array of diffraction grating waveguides is incorporated into wells of a microplate (JP Patent Publication (Kohyo) No. 2007-501432 A). That is to say, if such diffraction grating waveguides are aligned in the form of an array at the bottoms of wells of a microplate, the screening of a drug or chemical substance can be carried out at a high throughput.

In order to detect physiologically active substances existing on the upper layer (detection area) of a diffraction grating waveguide, the diffraction grating waveguide detects incident light and reflected light, so as to detect changes in refractive properties. For this purpose, one or more light sources (e.g. laser or diode) and one or more detectors (e.g. a spectrometer, a CCD camera, or other light detectors) can be used. As a method of measuring changes in refractive indexes, there are two different operational modes, namely, spectroscopy and an angle method. In spectroscopy, broadband beam used as incident light is transmitted to a diffraction grating waveguide, and reflected light is gathered, followed by a measurement with a spectrometer, for example. By observing the spectrum position of a resonant wavelength (peak), changes in refractive indexes on the surface of the diffraction grating waveguide or a periphery thereof, namely, a bond can be measured. On the other hand, in an angle method, light of a nominally single wavelength is gathered such that it generates a certain range of irradiation angle, and it is directed into the diffraction grating waveguide. The reflected light is measured with a CCD camera or other types of light detectors. By measuring the position of a resonance angle reflected by the diffraction grating wavelength, changes in refractive indexes on the surface of the diffraction grating waveguide or a periphery thereof, namely, a bond can be measured.

The present invention will be further specifically described in the following examples. However, the examples are not intended to limit the scope of the present invention.

### EXAMPLE

Experiments were conducted using the following devices.

A biosensor 10 as a screening device according to this embodiment is a so-called surface plasmon sensor that measures interaction between a physiologically active substance D and a compound using surface plasmon generated on the surface of a metal film. In this embodiment, this biosensor 10 is used for screening a compound that specifically binds to the physiologically active substance D among a large amount of compound A.

As shown in Figure 1, the biosensor 10 includes a tray-holding portion 12, a conveying portion 14, a container table 16, a liquid suction/discharge portion 20, an optical measurement portion 54, and a controller 60.

The tray-holding portion 12 is constituted to include a table 12A and a belt 12B. The table 12A is provided to the belt 12B traversed in the arrow-marked direction Y and can be move in the arrow-marked direction Y by rotation of the belt 12B. Two trays T are positioned and mounted on the table 12A. Eight sensor sticks 40 are stored in the trays T. The sensor stick 40 is a tip to which the physiologically active substance D is immobilized and will be described in detail below. A lifting mechanism 12D for lifting the sensor sticks 40 to the position of a stick-holding member 14C, which is described below, is disposed under the table 12A.

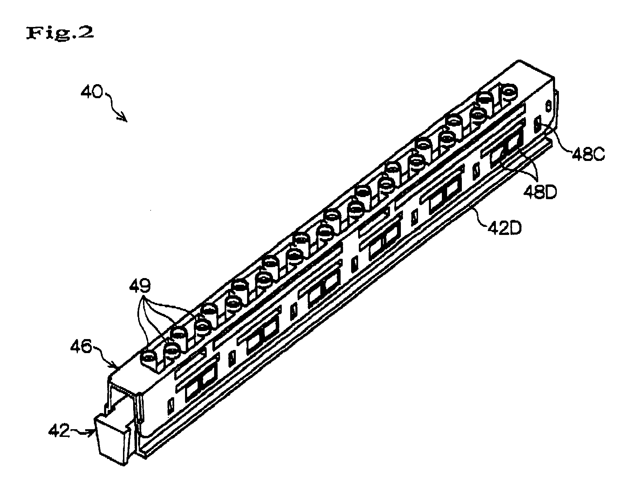

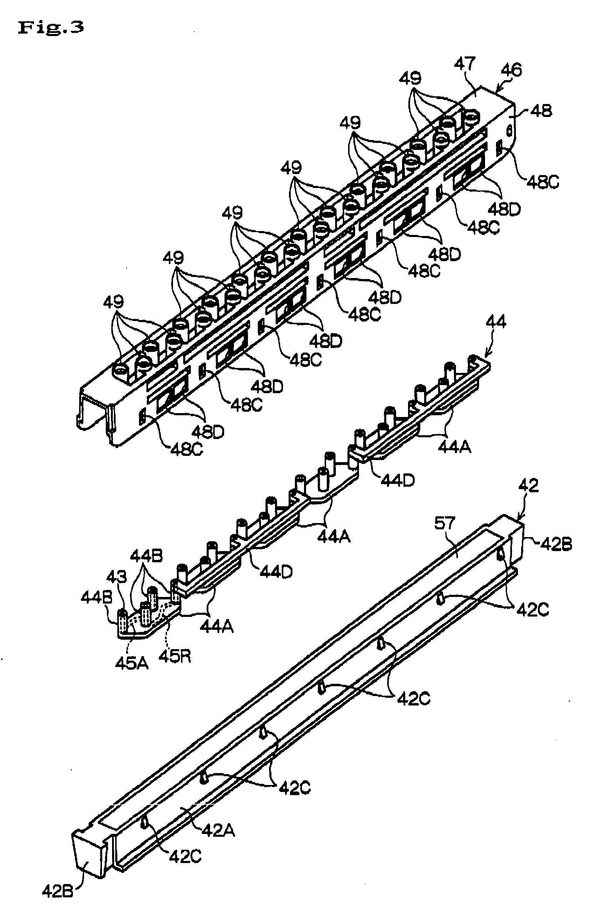

As shown in Figs. 2 and 3, the sensor stick 40 is constituted to include a dielectric block 42, a channel member 44, and a holder member 46.

The dielectric block 42 is made of, for example, a resin transparent to a light beam and includes a rodlike prism portion 42A having a trapezoidal cross section and held portions 42B that are integrally formed with the prism portion 42A at both ends of the prism portion 42A. A metal film 57 is disposed on the broader surface of the parallel faces of the prism portion 42A. The dielectric block 42 functions as a prism. In the measurement with the biosensor 10, a light beam enters one of the non-parallel opposing faces of the prism portion 42A, and the light beam is totally reflected at the interface with the metal film 57 and emitted from the other face of the non-parallel opposing faces.

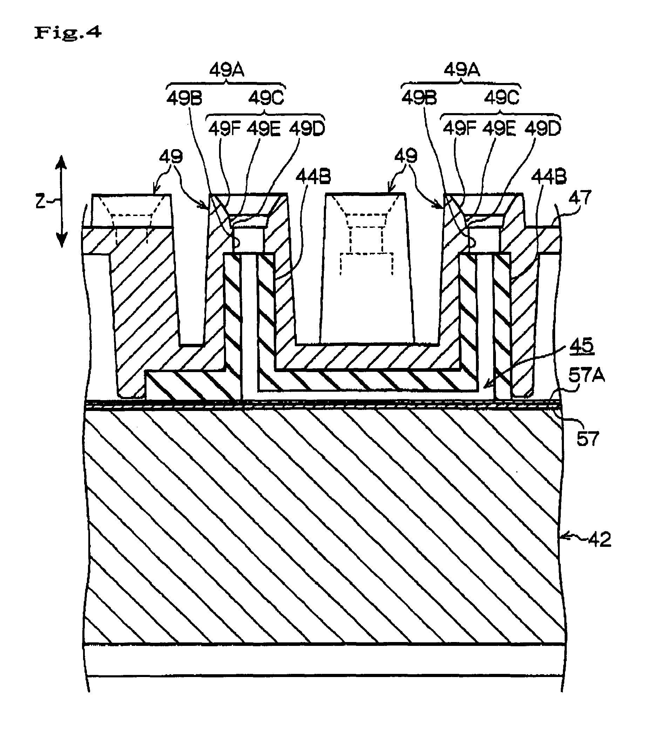

As shown in Figure 4, a linker layer 57A is formed on the surface of the metal film 57. The linker layer 57A functions as a layer for immobilizing a physiologically active substance D on the linker layer 57A.

The prism portion 42A is provided with engaging convex portions 42C that are engaged with the holder member 46 is provided at the both sides thereof along the edges of the upper side. Furthermore, at the lower side of the prism portion 42A, flanges 42D that are engaged with conveying rails (not shown) are provided along the side edges.

As shown in Figure 3, the channel member 44 includes six base portions 44A, and four cylindrical members 44B are set upright on each of the base portions 44A. In each three base portions 44A, one of the four cylindrical members 44B that are set upright on each base portion 44A is connected to cylindrical members 44B of the other two base portions 44A at the top with a connecting member 44D. The channel member 44 is made of a flexible, elastic, transformable material, for example, an amorphous polyolefin elastomer.

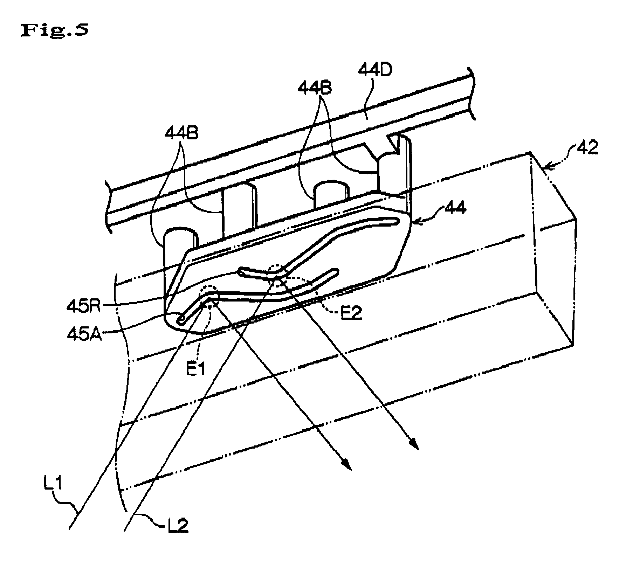

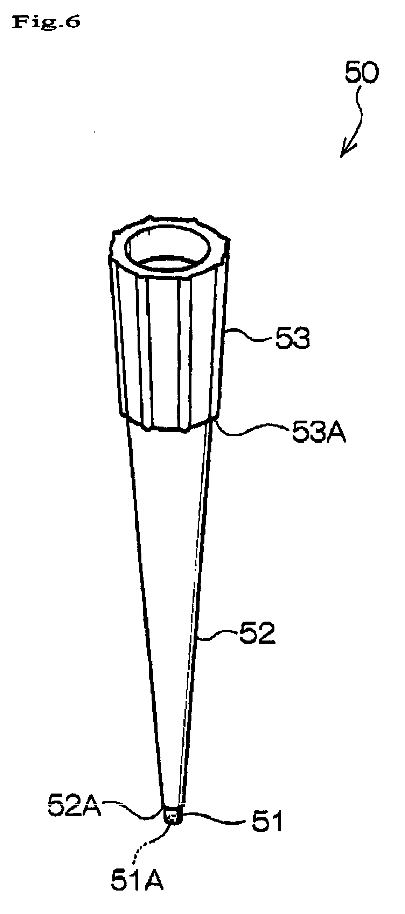

As shown in Figs. 5 and 6, the base portion 44A is provided with two channel grooves 44C having approximately an S-shape at the bottom side. Each end of the channel grooves 44C is connected to a hollow portion of the corresponding cylindrical member 44B. The bottom of the base portion 44A is adhered to the upper face of the dielectric block 42. The space formed between the channel groove 44C and the upper face of the dielectric block 42 and the above hollow portion constitute a liquid channel 45. Each of the base portions 44A is provided with two liquid channels 45. In each liquid channel 45, an inlet 43 of the liquid channel 45 is constituted at the upper face of the cylindrical member 44B.

Here, one of the two liquid channels 45 is used as a measurement channel 45A, and the other liquid channel 45 is used as a reference channel 45R. Measurement is conducted under a condition that a physiologically active substance D is immobilized on the metal film 57 of the measurement channel 45A, but is not immobilized on the metal film 57 of the reference channel 45R. As shown in Figure 5, light beams L1 and L2 enter the measurement channel 45A and the reference channel 45R, respectively. The light beams L1 and L2 irradiate the curvatures of the S-shaped channels disposed near the center of the base portion 44A. Hereinafter, the irradiation region of the light beam L1 in the measurement channel 45A is referred to as a measurement region E1, and the irradiation region of the light beam L2 in the reference channel 45R is referred to as a reference region E2. The reference region E2 is a region for conducting measurement for compensation of date obtained from the measurement region E1 to which the physiologically active substance D is immobilized.

The holder member 46 has a long length and a lid-like shape with an upper face member 47 and two side plates 48. The side plates 48 are provided with engaging holes 48C that are engaged with the engaging convex portions 42C of the dielectric block 42 and windows 48D at the portions corresponding to the light paths of the light beams L1 and L2. The holder member 46 is fit with the dielectric block 42 by engaging the engaging holes 46C and the engaging convex portions 42C. The channel member 44 is integrally formed with the holder member 46 as described below and is disposed between the holder member 46 and the dielectric block 42.

The upper face member 47 is provided with receiving portions 49 at the positions corresponding to the cylindrical members 44B of the channel member 44. As shown in Figure 4, the receiving portions 49 are approximately cylindrical, and the lower hollow portions thereof are connected to the cylindrical members 44B. Furthermore, a dent portion 49A that is in communication with the inlet 43 at 4 position upper than above hollow cylindrical member 44B is constituted. Into the dent portion 49A, a pipette tip 50 is inserted.

The holder member 46 is made of a material harder than that of the channel member 44, such as crystalline polyolefin.

As shown in Figure 6, the pipette tip 50 has approximately a circular cone shape and is constituted to include an end portion 51, a body portion 52, and a holder portion 53. The end portion 51 is cylindrical and has an opening 51A for ejecting or sucking a liquid at the end in the inserting direction. The body portion 52 has a circular cone shape having an external circumference larger than that of the end portion 51 and has an external circumferential bump 52A at the junction with the end portion 51. The external circumferential bump 52A is tapered toward the end portion 51. The holder portion 53 has an external circumference larger than that of the body portion 52 and has a holder bump 53A at the junction with the body portion 52. The holder bump 53A is a portion that is used when the pipette tip 50 is held in a pipette tip stocker (not shown) having an upper plate provided with a holding hole.

The dent portion 49A of the receiving portion 49 is constituted by being surrounded by a first inner wall 49B at the channel member 44 side and a second inner wall 49C. The first inner wall 49B has a length in the direction Z in which the pipette tip 50 is inserted slightly longer than that of the end portion 51 of the pipette tip 50 and an external diameter slightly larger than that of the end portion 51 and has a shape along the end portion 51.

The second inner wall 49C is constituted by an internal circumferential bump 49D at the junction with the first inner wall 49B, a central inner wall 49E adjacent to the internal circumferential bump 49D, and an upper inner wall 49F at the uppermost portion. The internal circumferential bump 49D is sequential to the first inner wall 49B and is tapered along the outer circumferential bump 52A so that the diameter is enlarged toward the upper side of the pipette tip 50. The central inner wall 49E is sequential to the internal circumferential bump 49D and is tapered so that the diameter is enlarged toward the upper side of the pipette tip 50. The upper inner wall 49F is sequential to the central inner wall 49E and is tapered so that the diameter is further enlarged toward the upper side of the pipette tip 50.

The holder member 46 and the channel member 44 are integrally formed by molding a combination of different materials in a single die, by so-called two-color molding (double molding).

As shown in Figure 1, the conveying portion 14 of the biosensor 10 is constituted to include an upper guide rail 14A, a lower guide rail 14B, and a stick-holding member 14C. The upper guide rail 14A and the lower guide rail 14B are disposed over the tray-holding portion 12 and the optical measurement portion 54 so as to be horizontal to the arrow-marked direction X that is orthogonal to the arrow-marked direction Y. The stick-holding member 14C is fit to the upper guide rail 14A. The stick-holding member 14C can hold the held portions 42B at both ends of the sensor stick 40 and can move along the upper guide rail 14A. An engaging groove 42E of the sensor stick 40 held by the stick-holding member 14C is engaged with the lower guide rail 14B and thereby the stick-holding member 14C can move in the arrow-marked direction X to convey the sensor stick 40 to the measurement portion 56 on the optical measurement portion 54. Furthermore, the measurement portion 56 is provided with a pushing member 58 for holding down the sensor stick 40 during a measurement process. The pushing member 58 can move in the Z direction by a driving mechanism (not shown) and presses the sensor stick 40 positioned at the measurement portion 56 from the upper side.

An analyte solution plate 17, a buffer solution storage container 18, and a liquid waste container 19 are placed on the container table 16. The analyte solution plate 17 is partitioned into a matrix and stores various types of analyte solutions. The buffer solution storage container 18 is constituted by a plurality of containers and stores a plurality of types of buffer solutions having different refraction indices. The buffer solution storage container 18 is provided with an opening K into which the pipette tip 50 can be inserted. The opening K will be described below. The liquid waste container 19 is constituted by a plurality of containers and is provided with an opening K into which the pipette tip 50 can be inserted, as in the buffer solution storage container.

As shown in Figure 1, the liquid suction/discharge portion 20 is constituted to include a head 24 and suction/discharge-driving portion 26. The head 24 can move in the arrow-marked direction Y (refer to Figure 1) along a convey rail (not shown). The head 24 can also move in the vertical direction (the arrow-marked direction Z) by a driving mechanism (not shown) inside the head 24.

A liquid is supplied to the liquid channel 45 with the head 24 by inserting two pipette tips 50 to two openings, respectively, of one liquid channel 45, and ejecting a liquid with one of the pipette tips 50 and, at the same time, sucking the liquid in the liquid channel 45 with the other pipette tip 50.

In this embodiment, a liquid is supplied to the sensor stick 40 with the pipette tip 50, but may be supplied using a liquid-conveying pump, instead of the pipette tip, being provided with an injection tube of which one end is connected to the solution plate and the other end can be connected to the sensor stick 40.

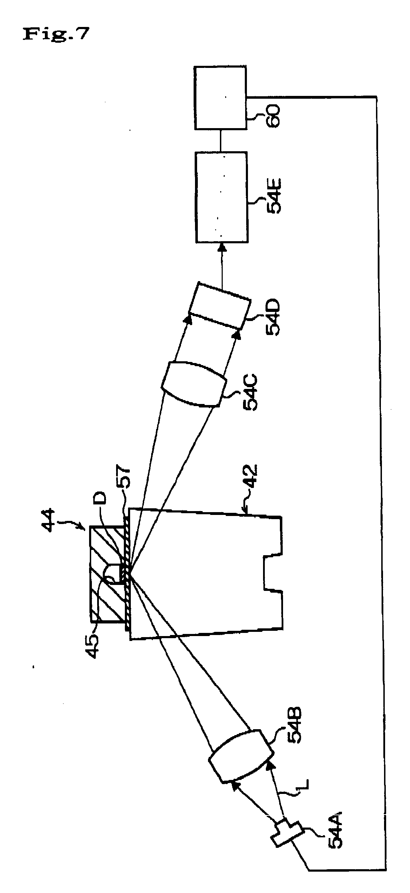

As shown in Figure 7, the optical measurement portion 54 is constituted to include a light source 54A, a first optical system 54B, a second optical system 54C, a light-receiving portion 54D, and a signal-processing portion 54E. The light source 54A emits a light beam L in a diverged state. The light beam L is split into two light beams L1 and L2 through the first optical system 54B. The light beams L1 and L2 enter the measurement region E1 and the reference region E2, respectively, of the dielectric block 42 disposed in the measurement portion 56. In the measurement region E1 and the reference region E2, each of the light beams L1 and L2 includes components having various incident angles to the interface of the metal film 57 and the dielectric block 42 and incidents on the interface at an angle larger than the total reflection angle. The light beams L1 and L2 are totally reflected at the interface of the dielectric block 42 and the metal film 57. The totally reflected light beams L1 and L2 are also reflected with various reflection angle components. These totally reflected light beams L1 and L2 are received by the light-receiving portion 54D through the second optical system 54C and are each photoelectrically converted so that an optical detection signal is output to the signal-processing portion 54E. In the signal-processing portion 54E, predetermined processing is conducted based on the input optical detection signals to determine measurement date G1 of the measurement region E1 and reference data G2 of the reference region E2. These measurement data G1 and the reference data G2 are output to the controller 60.

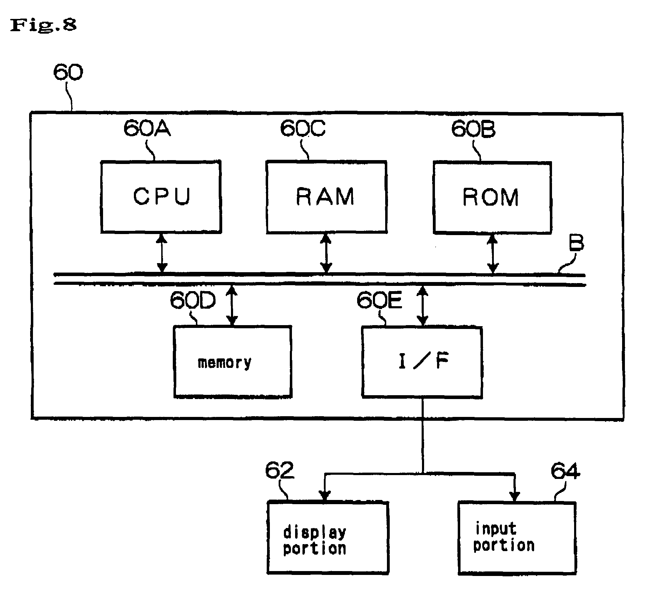

The controller 60 controls the entire biosensor 10 and, as shown in Figure 7, is connected to the light source 54A, the signal-processing portion 54E, and a driving system (not shown) of the biosensor 10. As shown in Figure 8, the controller 60 includes CPU 60A, ROM 60B, RAM 60C, memory 60D, and interface I/F 60E that are connected to each other through buses B and is connected to a display portion 62 for displaying various types of information and an input portion 64 for inputting various types of indication and information.

The memory 60D records various programs for controlling the biosensor 10 and various types of data.

### [Example]

### (1) Preparation of silicon oxide-surface substrate

A dielectric block substrate was prepared by injection molding of Zeonex (manufactured by Nippon Zeon Co., Ltd.). A chromium film with a thickness of 2 nm was formed on the dielectric block substrate and a gold film with a thickness of 50 nm was formed on the chromium film by sputtering using a sputtering apparatus (Model SH-550: manufactured by Ulvac Co.) for a 6-inch parallel flat plate. Sequentially, a silicon oxide film with a thickness of 20 nm was formed on the gold film by sputtering.

### (2) Preparation of silane coupling agent-surface substrate

An ethanol/hydrochloric acid (10 mN) (9/1: volume ratio) solution containing 0.1% 3-aminopropyltriethoxysilane (manufactured by Wako Pure Chemical Industries, Ltd.) was brought into contact with the gold-surface substrate prepared in the above (1), and surface treatment was conducted at 50°C for 12 minutes. Then, the substrate was washed with a 10% ethanol aqueous solution three times.

### (3) Preparation of CMD-surface substrate

Ten milliliters of an aqueous solution containing 1-ethyl-2,3-dimethylaminopropyl carbodiimide (2 × 10⁻⁵ M) and N-hydroxysuccinimide (5 × 10⁻⁵ M) were added to 10 g of a 1 % carboxymethyl dextran (CMD: manufactured by Meito Sangyo Co., Ltd., molecular weight: 1,000,000, substitution degree: 0.59) solution (the amount of carboxyl group: 2.8 × 10⁻⁴ mol). The mixture was stirred at room temperature for 1 hour. One milliliter of the stirred solution was dropwise added onto the substrate prepared in the above (2), and the substrate was fixed on the inner cup of a spin coater (Model 408 (Special): manufactured by Nanometric Technology Inc.) having a sealed type inner cup such that the tangential line direction of a circular arc is in the long axis direction of the substrate at a position of a radius of 135 mm from the rotation center. A thin film of an actively esterified carboxymethyl dextran was spin-coated on the silane coupling agent-surface substrate by rotating the spin coater at 1000 rpm for 45 seconds. After the reaction at room temperature for 15 minutes, the substrate was immersed in a 1 N NaOH aqueous solution for 30 minutes and then washed with ultra-pure water 5 times to give a CMD-surface substrate.

### (4) Evaluation of substrate surface for non-specific adsorption inhibition

Non-specific adsorption of protein to the surface of a biosensor causes noise and is hence required to be reduced as much as possible. The prepared CMD-surface substrate was set to a surface plasmon resonance measuring apparatus and measured for non-specific adsorption of CGP-74514A hydrochloride (manufactured by Sigma). A 10% by volume of DMSO-containing HBS-N buffer solution (manufactured by Biacore) was added onto the substrate, and the substrate was left standing for 20 minutes. Subsequently, a CGP-74514A hydrochloride solution (10 µM, 10% by volume of DMSO-containing HBS-N buffer solution) was added onto the substrate, and the substrate was left standing for 10 minutes. The composition of the HBS-N buffer was 0.01 mol/L of HEPES (N-2-hydroxyethylpiperazine-N'-2-ethanesulfonic acid) (pH 7.4) and 0.15 mol/L of NaCl. The amount of a change in the resonance signal (RU value) after the leaving standing for 10 minutes was used as the amount of non-specific adsorption of CGP-74514A hydrochloride, The measurement results are shown in Table 1.
From the results shown in Table 1, it was confirmed by the surface plasmon resonance that, in a biosensor using the substrate according to the present invention, the non-specific adsorption of a low molecular compound was significantly small.

### (5) Evaluation of substrate surface for physiologically active substance immobilizing ability

The CMD-surface substrate prepared in the above (3) was set to a surface plasmon resonance measuring apparatus and was brought into contact with a solution mixture of 1-ethyl-2,3-dimethylaminopropyl carbodiimide (200 mM) and N-hydroxysuccinimide (50 mM) for 30 minutes and then washed with a 50 mM acetic acid buffer (pH 4.5, manufactured by Biacore). Then, the substrate was brought into contact with a Protein A (manufactured by Nakalai Tesque) solution (100 µg/mL, 50 mM acetic acid buffer, pH 4.5) for 30 minutes and then washed with a 50 mM acetic acid buffer (pH 4.5).

Furthermore, the substrate was brought into contact with an ethanolamine-HCl solution (1 M, pH 8.5) for 30 minutes and then washed with a 50 mM acetic acid buffer (pH 4.5) to block the remaining activated COOH group that had not reacted with Protein A.

Furthermore, the substrate was brought into contact with a NaOH aqueous solution (10 mM) for 1 minute and then washed with an HBS-EP buffer (HEPES (N-2-hydroxyethylpiperazine-N'-2-ethanesulfonic acid): 0.01 mol/L (pH 7.4), NaCl: 0.15 mol/L, EDTA: 0.003 mol/L, surfactant P20: 0.005% by weight, manufactured by Biacore) to remove Protein A that was non-specifically adsorbed onto the spin-coat tip surface. The difference of a resonance signal (RU value) in an HBS-EP buffer after the contact with the NAOH aqueous solution and that before the contact with the Protein A solution was used as the amount of immobilized Protein A. The measurement results are shown in Table 1.

From the results shown in Table 1, it was confirmed by the surface plasmon resonance that, in a biosensor using the substrate according to the present invention, a physiologically active substance was satisfactorily immobilized.

### [Comparative Example 1]

### (1) Preparation of silane coupling agent-surface substrate (comparison)

A silane coupling agent-surface substrate as a comparative example was prepared by the same procedure as in Example (2) except that (CH₃O)₃SiC₃H₆SCH₂CH₂COOH was used instead of 3-aminopropyltriethoxysilane to the silicon oxide-surface substrate prepared in Example (1).

The substrate was evaluated for the properties as in Example (4) and (5). The results are shown in Table 1.

### [Comparative Example 2]

A dielectric block substrate was prepared by injection molding of Zeonex (manufactured by Nippon Zeon Co., Ltd.). A chromium film with a thickness of 2 nm was formed on the dielectric block substrate and a gold film with a thickness of 50 nm was formed on the chromium film by sputtering using a sputtering apparatus (Model SH-550: manufactured by Ulvac Co.) for a 6-inch parallel flat plate. This dielectric block substrate was immersed in a 1 mM aqueous solution of 6-amino-1-octanethiol, hydrochloride (manufactured by Dojindo Laboratories) at 40°C for 1 hour and then washed with ultra-pure water five times.

A CMD-surface substrate of Comparative Example 2 was prepared by the same method as in Example 1 (3).

The substrate was evaluated for properties as in Example (4) and (5). The results are shown in Table 1.

**Table 1**

| | Non-specific adsorption of low molecular compound | Amount of immobilized physiologically active substance |
|---|---|---|
| Example (the present invention) | 12 RU | 16000 RU |
| Comparative Example 1 | 97 RU | 3600 RU |
| Comparative Example 2 | 35 RU | 17000 RU |

## Claims

1. A biosensor substrate which comprises an inorganic oxide film and a hydrophilic polymer having a physiologically active substance-immobilizing group on the substrate, wherein the hydrophilic polymer is bound to the inorganic oxide film through a low molecular compound.

2. The biosensor substrate of claim 1 wherein the binding is a covalent bond.

3. The biosensor substrate of claim 2 wherein the low molecular compound is a compound represented by the following formula:
X-L(Y)n
wherein X represents a functional group that can bind to an inorganic oxide and is selected from the group consisting of -Si(OCH₃)₃, -Si(OC₂H₅)₃, -Si(OC₃H₇)₃, and -Si(OC₄H₉)₃; Y represents a functional group that binds to a hydrophilic polymer and is selected from the group consisting of -OH, -SH, -COOH, -NH₂, -NR¹R² (wherein R¹ and R² each independently represent a hydrogen atom or a lower alkyl group), -CHO, a glycidoxy group, a methacryloxy group, a mercapto group, a vinyl group, and an epoxy group; when n is not 1, Y's may be the same or different; L represents a single bond or a group selected from the group consisting of an alkylene group, a phenylene group, -NH-, -CO-NH-, -O-, -S-, polyethylene oxide, polypropylene oxide, fatty acid ester, and a combination thereof, and may include -OH, -SH, -COOH, -NH₂, -NR¹R² (wherein R¹ and R² each independently represent a hydrogen atom or a lower alkyl group), or -CHO in the molecule; and n represents an integer of 1 to 5.

4. The biosensor substrate of claim 1 wherein the inorganic oxide is selected from the group consisting of silicon oxide, titanium oxide, zirconium oxide, and a mixture thereof.

5. The biosensor substrate of claim 1 wherein the inorganic oxide film has a thickness of 0.1 to 500 nm.

6. The biosensor substrate of claim 1 wherein the inorganic oxide film is formed on a layer of gold, silver, or platinum.

7. The biosensor substrate of claim 1 wherein the physiologically active substance-immobilizing group is selected from the group consisting of -OH, -SH, -COOH, -NR¹R² (wherein R¹ and R² each independently represent a hydrogen atom or a lower alkyl group), -CHO, -NR³NR¹R² (wherein R¹, R², and R³ each independently represent a hydrogen atom or a lower alkyl group) -NCO, -NCS, an epoxy group, a vinyl group, a biotinyl group, biotin-binding protein, digoxigenin, and an anti-digoxigenin antibody.

8. A biosensor which comprises the biosensor substrate of claim 1.
